# NEW EUROPEAN PATENT SPECIFICATION

(11) **EP 1 181 047 B2**
(45) Date of publication and mention of the opposition decision: **28.10.2009**
(45) Mention of the grant of the patent: 16.04.2003
(21) Application number: 00940741.2
(22) Date of filing: 07.06.2000
(51) Int. Cl.: A61K 38/46, A61K 35/74, A61P 1/00, A61P 17/00, A61P 25/00, A61P 35/00

(54) **COMPOSITION COMPRISING ALKALINE SPHINGOMYELINASE OF BACTERIAL ORIGIN FOR USE AS A DIETETIC PREPARATION, FOOD SUPPLEMENT OR PHARMACEUTICAL PRODUCT**
ZUSAMMENSETZUNG ENTHALTEND ALKALISCHE SPHINGOMYELASE VON BAKTERIELLEN URSPRUNG ZUM GEBRAUCH ALS DIÄTETISCHE ZUSAMMENSETZUNG, NAHRUNGSERGÄNZUNGSMITTEL ODER PHARMAZEUTISCHES PRODUKT
COMPOSITION RENFERMANT DE LA SPHINGOMYELINASE ALCALINE D'ORIGINE BACTÉRIENNE UTILISEE COMME PREPARATION DIETETIQUE, COMPLEMENT ALIMENTAIRE OU PRODUIT PHARMACEUTIQUE

(30) Priority: 09.06.1999 IT RM990376
(43) Date of publication of application: 27.02.2002
(73) Proprietor: ACTIAL Farmaceutica Lda., Funchal, Madeira (PT)
(72) Inventor: DE SIMONE, Claudio, I-00040 Ardea (IT)
(74) Representative: Cavattoni, Fabio
(86) International application number: PCT/IT2000/000230
(87) International publication number: WO 2000/074712

(56) References cited:
- EP-A2- 0 015 552
- WO-A-98/00035
- WO-A-98/22082
- WO-A-99/42568
- U. SJOQVIST ET AL.: "Chronic colitis with dysplasia is associated with a reduction of alkaline sphingomyelinase activity." GASTROENTEROLOGY, vol. 116, no. 4 part 2, April 1999 (1999-04), page A505 XP000971701 New York, US
- RUI-DONG DUAN: "Sphingomyelin hydrolysis in the gut and clinical implications in colorectal tumorigenesis and other gastrointestinal diseases." SCANDINAVIAN JOURNAL OF GASTROENTEROLOGY, vol. 33, no. 7, July 1998 (1998-07), pages 673-683, XP000971699 Oslo, SE cited in the application
- LENA NYBERG: 'Digestion and Absorption of Sphingomyelin from Milk', 1998, ISBN 91-628-2963-7
- LENA NYBERG ET AL. BIOCHIMICA ET BIOPHYSICA ACTA vol. 1300, 1996, pages 42 - 48
- DUAN ET AL.: 'Alkaline sphingomyelinase activity in rat gastrointestinal tract: distribution and characteristics' BIOCHIMICA ET BIOPHYSICA ACTA vol. 1259, 1995, pages 49 - 55
- DUAN ET AL.: 'Purification of a Newly Indentified Alkaline Sphingomyelinase in Human Bile and Effects of Bile Salts and Phosphatidylcholine on Enzyme Activity' HEPATOLOGY vol. 26, no. 4, 1997,
- ERIK HERTERVIG: 'Alkaline Sphingomyelinase A Potential Inhibitor in Colorectal Carcinogenesis', 2000, ISBN 91-628-4191-2
- E. HERTERVIG ET AL.: 'Purified intestinal alkaline sphingomyelinase inhibits proliferation without inducing apoptosis in HT-29 colon carcinoma cells' J CANCER RES CLIN ONCOL vol. 129, 2003, pages 577 - 582
- DUAN ET AL.: 'Purification, localization, and expression of human intestinal alkaline sphingomyelinase' JOURNAL OF LIPID RESEARCH vol. 44, 2003,
- DUAN ET AL.: 'Distribution of Alkaline Sphingomyelinase Activity in Human Beings and Animals' DIGESTIVE DISEASES AND SCIENCES vol. 47, no. 9, September 1996, pages 1801 - 1806
- DUAN: 'Alkaline sphingomyelinase: An old enzyme with novel implications' BIOCHIMICA ET BIOPHYSICA ACTA vol. 1761, 2006, pages 281 - 291
- Soo et al. VSL#3 probiotic up-regulates mucosal alkaline sphingomyelinase: a mechanism implicated in the reduction of colon cancer
- Rossignol et al. Hemolytic activity of different strain of pseudomonas fluorescens
- SUEYOSHI ET AL.: 'Molecular Cloning and Expression of Mn2+-Dependent Sphingomyelinase/Hemolysin of an Aquatic Bacterium, Pseudomonas sp. Strain TK4' JOURNAL OF BACTERIOLOGY vol. 184, no. 2, January 2002, pages 540 - 546

## Description

The present invention relates to dietetic, nutrient or pharmaceutical compositions containing alkaline sphingomyelinase

Consequently, these compositions may be in the form of and may act as food supplements, dietetic bases or medicinal preparations proper, according to whether they are intended to act as bases or prophylactic treatments or as therapeutic preparations proper, depending on the particular individuals for whom the composition is intended.

Three different types of sphingomyelinase (SMase) have so far been identified.

There is an acidic sphingomyelinase, which is a lysosomal enzyme (with an optimum pH of 4.5-5), deficiency of which causes Niemann-Pick disease, and there is a neutral sphingomyelinase, with an optimum pH of 7.5, for which two iso-forms have been described. One of these iso-forms is located in the cytoplasmic membrane and depends on magnesium, while the other is contained in the cytosol and is independent of cations. Both the acidic and the neutral sphingomyelinase are found in many tissues and cells and are ubiquitous enzymes, regulating numerous cell functions.

The third type is called alkaline sphingomyelinase, because it is mainly active at pH 9. It is independent of magnesium and has been found both in intestinal brush borders and in the bile. Alkaline sphingomyelinase does not occur in the stomach, duodenum or pancreas but it is found in the intestine, especially in the distal part of the jejunum. A marked alkaline sphingomyelinase activity has also been observed in the colon and the rectum. High levels of alkaline sphingomyelinase are also found in the bile, but this seems to be peculiar to human beings. This twofold source of sphingomyelinase makes human beings very efficient in comparison with other creatures as regards the hydrolysis of sphingomyelin (SM) introduced via the diet. It has hitherto been thought that alkaline sphingomyelinase cannot be produced by intestinal bacteria, because no differences have been found between conventional and germ-free animals [see R.D. Duan, Scand. J. Gastroenterology, 33 (1998) pp. 673-683].

Apart from the alkaline sphingomyelinase that is present in the intestine and that present in the bile, no other alkaline sphingomyelinases are known that could be used to produce compositions intended for nutritional, dietetic or strictly therapeutic use. Moreover, acidic and neutral sphingomyelinase cannot be employed owing to their differing characteristics (see the following table).

**Table**

| Location | Acidic SMase lysosomes | Neutral SMase cytoplasmic membrane | Alkaline SMase human intestine and bile |
|---|---|---|---|
| Optimum pH | 5.5 | 7.4 | 9 |
| Mg⁺⁺-dependence | No | Yes | No |
| Trypsin resistance | No | No | Yes |
| Thermal stability | < 40°C | - | < 50-60°C |
| Substrate | endocytic SM | membrane SM | SM in food |

The use of sphingomyelinase for cosmetic and dermatological purposes is already known.

Japanese Patent No. 63 216,813 describes cosmetic compositions that contain sphingomyelinase and are intended for counteracting the physiological decrease of this enzyme that occurs in the skin on ageing, and for promoting its transformation into ceramide which, in turn, has a beneficial moisturizing effect on the epidermis.

International Patent Application PCT WO 98/22,082 describes the use of sphingomyelinase for the preparation of dermatological compositions suitable for treating skin disorders such as dermatitis, psoriasis, ichthyosis and similar conditions. Furthermore, this PCT application describes the preparation of sphingomyelinase from strains of Gram-negative bacteria, Gram-positive bacteria and lactic acid bacteria, with clear advantages over the previously known processes, which use the organs of higher animals, such as the brain and liver, as starting materials.

It has now been found, surprisingly, that some bacteria possess high levels of alkaline sphingomyelinase, and that their ingestion can be beneficial for the host. These bacteria can be ingested live or in the form of extracts, provided that these are enzymatically active, possibly in combination with other bacteria such as lactic acid bacteria, with SM and/or with foods containing SM.

One of the objects of the present invention is therefore to provide a dietetic, nutrient or pharmaceutical composition that comprises alkaline sphingomyelinase in an amount that is sufficient to exert a dietetic, nutritional or therapeutic effect in an individual who needs it.

In particular, this composition is suitable for the prevention and/or treatment of disorders connected with intestinal development, cancerous processes, disorders of the immune response, inflammatory and apoptosic processes of the intestine and its associated structures, disorders connected with cholesterol synthesis, disorders due to the hydrophobic nature of the surfaces of the gastrointestinal tract, allergic disorders of the gastro-intestinal tract, disorders relating to digestive processes, inflammatory intestinal diseases, polyposis, in particular familial polyposis, hypercholesterolaemia, infections with *Helicobacter pylori,* disorders of neonatal growth, disorders connected with intestinal homeostasis and diseases of the central and peripheral nervous systems.

The composition is also useful for use in pediatric diets and/or in enteral alimentation. In pediatric diets the composition may be administered, for example, in combination with artificial milk, condensed milk, soybean milk, powdered milk, partially umanized milk and baby foods in general.

The composition contains alkaline sphingomyelinase of bacterial origin, and the bacteria containing the alkaline sphingomyelinase are chosen from amongst Gram-positive bacteria, Gram-negative bacteria and lactic acid bacteria, or from mixtures thereof.

More especially, the alkaline sphingomyelinase of the composition is obtained from lactic acid bacteria, and these are chosen from the group comprising *Lactobacillus acidophilus, Lactobacillus brevis, Lactobacillus buchneri, Lactobacillus casei, Lactobacillus catenaforme, Lactobacillus cellobiosus, Lactobacillus crispatus, Lactobacillus curvatus, Lactobacillus delbrueckii, Lactobacillus fermentum, Lactobacillus jensenii, Lactobacillus leichmannii, Lactobacillus minutus, Lactobacillus plantarum, Lactobacillus rogosae, Lactobacillus salivarius, Bifidobacterium adolescentis, Bifidobacterium angulatum, Bifidobacterium bifidum, Bifidobacterium breve, Bifidobacterium catenulatum, Bifidobacterium dentium, Bifidobacterium eriksonii, Bifidobacterium infantis, Bifidobacterium longum, Bifidobacterium plantarum, Bifidobacterium pseudocatenulatum, Bifidobacterium pseudolongum, Streptococcus lactis, Streptococcus raffinolactis* and *Streptococcus thermophilus.*

The particularly preferred strain amongst these lactic acid bacteria is *Lactobacillus brevis* CD2, filed on February 6, 1998 under access No. DSM 11,988 in the German Collection of Micro-organisms and Cell Cultures (DSM) in Braunschweig, Germany ("Deutsche Sammlung von Mikroorganismen und Zeilkulturen GmbH") under the Budapest Treaty, or mutants or derivatives thereof.

According to a preferred embodiment of the invention, the lactic acid bacteria are used in the composition as live, lyophilized or sonicated bacteria.

The composition preferably contains from 1 x 10² to 1 x 10¹³ CFUs of lactic acid bacteria per gram of composition.

A particularly preferred composition contains 200 x 10⁹ *Streptococcus thermophilus,* 150 x 10⁹ Bifidobacteria and 4 x 10⁹ *Lactobacillus acidophilus* per gram of composition.

The composition according to the invention can also contain bile acids, in particular ursodeoxycholic acid, pectin, sphingomyelin or its compounds, drugs or foods containing sphingomyelin, arginine deiminase, fatty acids, polyunsaturated fatty acids, non fermented sugars, in particular lactulose, cholesterol inhibitors, ceramidase inhibitors, protease inhibitors, immunomodulators, anti-carcinogenic agents, vitamins, growth factors, surfactants, cereals, fibre, emulsifiers, stabilizers, lipids, antioxidants, preservatives, free-radical neutralizers and/or vasoprotectors.

The composition of the invention can be administered orally as a food supplement or orally or parenterally as a drug.

The invention also relates to the use of alkaline sphingomyelinase of bacterial origin, and the bacteria containing the alkaline sphingomyelinase are chosen from amongst Gram-positive bacteria, Gram-negative bacteria and lactic acid bacteria, and from mixtures thereof for the preparation of a dietetic, nutrient or pharmaceutical composition suitable for the prevention and/or treatment of disorders connected with intestinal development, cancerous processes, disorders of the immune response, inflammatory and apoptosic processes of the intestine and its associated structures, disorders connected with cholesterol synthesis, disorders due to the hydrophobic nature of the surfaces of the gastrointestinal tract, allergic disorders of the gastro-intestinal tract, disorders relating to digestive processes, inflammatory intestinal diseases, polyposis, in particular familial polyposis, hypercholesterolaemia, infections with *Helicobacter pylori,* disorders of neonatal growth, disorders connected with intestinal homeostasis and diseases of the central and peripheral nervous systems.

This composition is also useful for use in pediatric diets and/or in enteral alimentation. In pediatric diets the composition may be administered, for example, in combination with artificial milk, condensed milk, soybean milk, powdered milk, partially umanized milk and baby foods in general.

The alkaline sphingomyelinase used is of bacterial origin, and the bacteria containing it are chosen from amongst Gram-positive bacteria, Gram-negative bacteria and lactic acid bacteria, or from mixtures thereof.

More especially, the lactic acid bacteria used are chosen from the group comprising *Lactobacillus acidophilus, Lactobacillus brevis*, *Lactobacillus buchneri, Lactobacillus casei, Lactobacillus catenaforme, Lactobacillus cellobiosus, Lactobacillus crispatus, Lactobacillus curvatus, Lactobacillus delbrueckii, Lactobacillus fermentum, Lactobacillus jensenii, Lactobacillus leichmannii, Lactobacillus minutus, Lactobacillus plantarum, Lactobacillus rogosae, Lactobacillus salivarius, Bifidobacterium adolescentis, Bifidobacterium angulatum, Bifidobacterium bifidum, Bifidobacterium breve, Bifidobacterium catenulatum, Bifidobacterium dentium, Bifidobacterium eriksonii, Bifidobacterium infantis, Bifidobacterium longum, Bifidobacterium plantarum, Bifidobacterium pseudocatenulatum, Bifidobacterium pseudolongum, Streptococcus lactis, Streptococcus raffinolactis* and *Streptococcus thermophilus.*

The particularly preferred strain amongst these lactic acid bacteria is *Lactobacillus brevis* CD2, filed on February 6, 1998 under access No. DSM 11,988 in the German Collection of Micro-organisms and Cell Cultures (DSM) in Braunschweig, Germany ("Deutsche Sammlung von Mikroorganismen und Zellkulturen GmbH") under the Budapest Treaty, or mutants or derivatives thereof.

According to a preferred embodiment of the invention, the lactic acid bacteria are used in the composition as live, lyophilized or sonicated bacteria.

The composition used preferably contains from 1 x 10² to 1 x 10¹³ CFUs of lactic acid bacteria per gram of composition.

A particularly preferred composition contains 200 x 10⁹ *Streptococcus thermophilus,* 150 x 10⁹ Bifidobacteria and 4 x 10⁹ *Lactobacillus acidophilus* per gram of composition.

The following experiments were carried out to confirm the presence and efficacy of alkaline sphingomyelinase in the bacteria according to the present invention. These experiments involved the detection of alkaline sphingomyelinase, the enzyme responsible for the formation of ceramide in human skin.

### Methods

### Assay of acidic, neutral and alkaline sphingomyelinase in lactic acid bacteria and in intestinal biopsy material

10 mg of lyophilized *Streptococcus thermophilus* bacteria were suspended in 500 µl of a buffer containing 50 mM Tris-HCl, pH 7.4, 10 mM MgCl₂, 2 mM EDTA, 5 mM DTT, 0.1 mM Na₃VO₄, 0.1 mM Na₂MoO₄, 30 mM p-nitrophenyl phosphate, 10 mM β-glycerophosphate, 750 mM ATP, 1 µM PMSF, 10 µM leupeptin, 10 µM pepstatin (from Sigma Chemical Co.) and 0.2% Triton X-100 (to assay the activity of neutral SMase) or 500 µl of 0.2% Triton X-100 (to assay the activity of acidic SMase). To assay the alkaline SMase, the bacteria and the (homogenized) intestinal biopsy material were suspended in a 0.25 M sucrose buffer containing 5 mM MgCl₂, 0.15 M KCl, 50 mM KH₂PO₄, 1 mM PMSF and 1 mM benzamidine (pH 7.4). The samples prepared in this way were then subjected to lysis by sonication (for 30 min during which, 10-sec "on" periods alternated with 10-sec "off" periods), using a Vibracell sonicator (Sonic and Materials Inc., Danbury, CT). The sonicated samples were then centrifuged for 30 min at 14,000 rpm at 4°C, the supernatant was removed, and the protein concentration was determined with a kit made by Bio-Rad Laboratories, (Richmond, CA).

To determine the neutral SMase, 100 µg of the sample were incubated for 2 hours at 37°C in a buffer (final volume: 50 µl) containing 50 mM Tris-HCl, 1 mM MgCl₂, pH 7.4, and 2.25 µl of [N-methyl-¹⁴C]-sphingomyelin (SM) (0.2 µCi/ml, specific activity: 56.6 mCi/mmol, Amersham).

To determine the activity of the acidic sphingomyelinase, 100 µg of the bacterial lysate were incubated for 2 hours at 37°C in a buffer (final volume: 50 µl) containing 250 mM sodium acetate, 1 mM EDTA, pH 5.0, and 2.25 µl of [N-methyl-¹⁴C]-SM.

To assay the alkaline SMase, the samples were added to 375 µl of Tris-EDTA buffer (pH 9) to a final volume of 0.4 ml, containing 50 mM Tris, 0.15 M NaCl 2 mM EDTA and a mixture of 3 mM bile salts with a TC : TDC : GC : GCDC molar ratio of 3 : 2 : 1.8 : 1. This mixture of bile salts had been found to possess the highest stimulatory effect on alkaline SMase. The addition of EDTA to the buffer served to inhibit the activity of neutral SMase, which is Mg⁺⁺-dependent with an optimum pH of 7.5. The ¹⁴C-SM was dissolved in ethanol, dried under nitrogen and suspended in the assay buffer, containing a mixture of 3% Triton X-100 and 3 mM bile salts.

The reaction was terminated by the addition of 2 ml of a 2:1 mixture of chloroform and methanol. The phospholipids were extracted and analysed on TLC plates, while the hydrolysis of the SM was quantified by autoradiography and liquid scintillation counting. The SMase present in the sonicated bacteria and in the intestinal biopsy material was expressed as pmol of SM hydrolysed per hour per milligram of protein.

### Activity of SMase from Streptococcus thermophilus

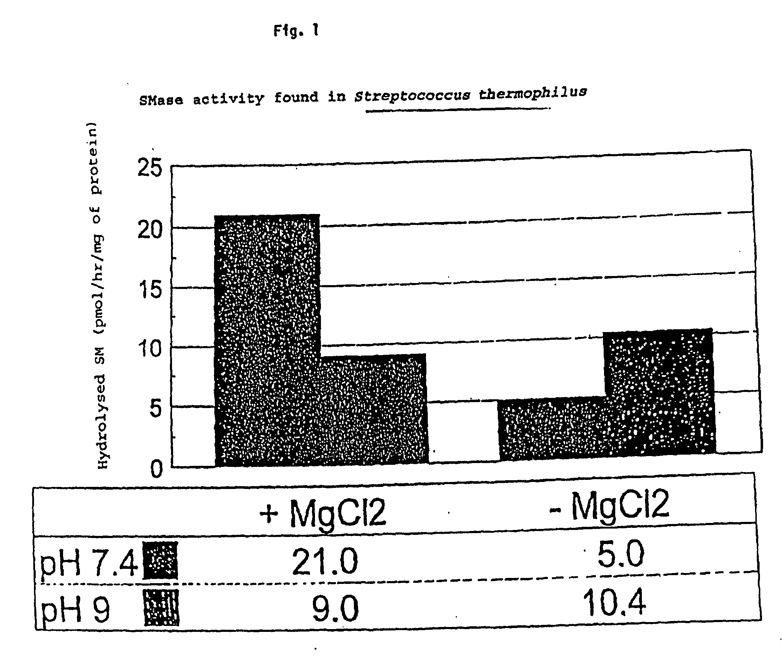

Figure 1 shows the activity levels of sphingomyelinase in sonicated lactic acid bacteria. No activity due to acidic SMase was found, but appreciable levels of both neutral and alkaline SMase were observed in the bacterial samples tested under the experimental conditions used (various pH values and with and without MgCl₂).

### Alkaline SMase found in intestinal biopsy material

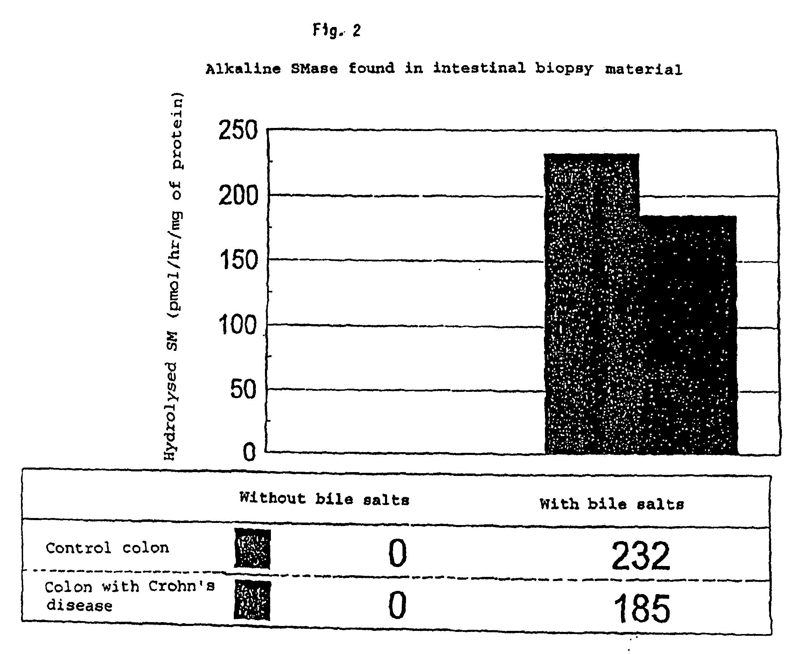

Figure 2 shows that the analysis of SMase activity in the intestinal biopsy samples showed a high activity of alkaline SMase of the kind dependent on bile salts, which could not be detected in the absence of bile salts. The levels of enzymatic activity in the tissues of a patient suffering from Crohn's disease showed a lower level of alkaline SMase than the control sample.

### Effect of Streptococcus thermophilus on intestinal alkaline SMase

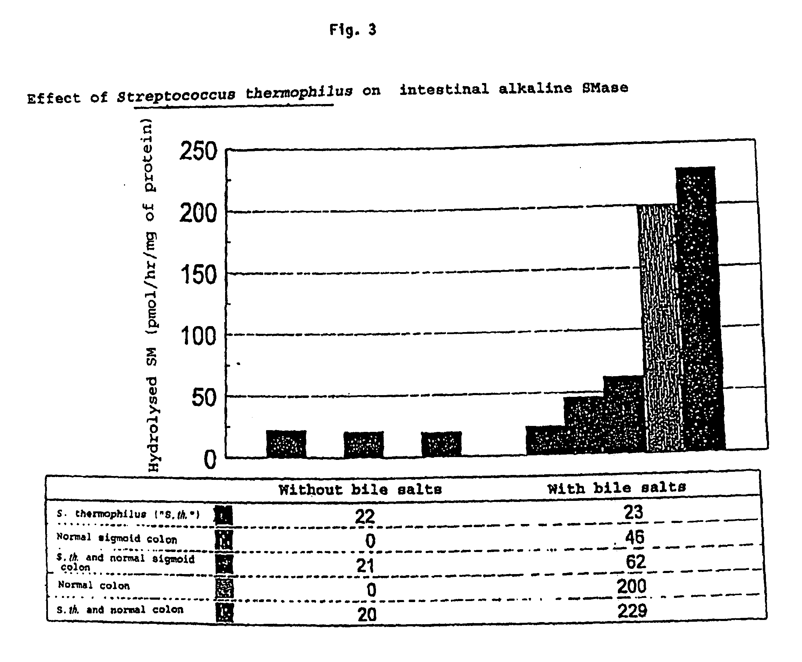

As shown in figure 3, the assay of the activity of SMase in the samples of *Streptococcus thermophilus,* under the experimental conditions used for the determination of intestinal SMase, showed that the bacterial enzyme was not affected by the presence or absence of bile salts. Furthermore, when the bacterial SMase activity and the intestinal SMase activity were tested simultaneously, the hydrolysis of SM increased additively. Similar results (not shown) were obtained with the *Lactobacillus brevis* CD2 strain, filed on February 6, 1998 under access No. DSM 11,988 in the German Collection of Microorganisms and Cell Cultures in Braunschweig, Germany ("Deutsche Sammlung von Mikroorganismen und Zellkulturen GmbH") under the Budapest Treaty, or mutants or derivatives thereof.

## Claims

1. Dietetic, nutrient or pharmaceutical composition, **characterized in that** it comprises alkaline sphingomyelinase of bacterial origin, and the bacteria containing the alkaline sphingomyelinase are chosen from amongst Gram-positive bacteria, Gram-negative bacteria and lactic acid bacteria, or from mixtures thereof; in an amount that is sufficient to exert a dietetic, nutritional or therapeutic effect in an individual who need it.

2. Composition according to claim 1, suitable for the prevention and/or treatment of disorders connected with intestinal development, cancerous processes, disorders of the immune response, inflammatory and apoptosic processes of the intestine and its associated structures, disorders connected with cholesterol synthesis, disorders due to the hydrophobic nature of the surfaces of the gastrointestinal tract, allergic disorders of the gastrointestinal tract, disorders relating to digestive processes, inflammatory intestinal diseases, polyposis, in particular familial polyposis, hypercholesterolaemia, infections with Helicobacter pylori, disorders of neonatal growth, disorders connected with intestinal homeostasis and diseases of the central and peripheral nervous systems.

3. Composition according to claim 1 or 2, suitable for use in pediatric diets.

4. Composition according to claim 1 or 2, suitable for use in enteral alimentation.

5. Composition according to claim 3, **characterized in that** the composition is administered, in combination with artificial milk, condensed milk, soybean milk, powdered milk, partially humanized milk and baby foods in general.

6. Composition according to any one of the preceding claims, **characterized in that** the lactic acid bacteria are chosen from the group comprising *Lactobacillus acidophilus, Lactobacillus brevis, Lactobacillus buchneri, Lactobacillus casei, Lactobacillus catenaforme, Lactobacillus cellobiosus, Lactobacillus crispatus, Lactobacillus curvatus, Lactobacillus delbrueckii, Lactobacillus fermentum, Lactobacillus jensenii, Lactobacillus leichmannii, Lactobacillus minutus, Lactobacillus plantarum, Lactobacillus rogosae, Lactobacillus salivarius, Bifidobacterium adolescentis, Bifidobacterium angulatum, Bifidobacterium bifidum, Bifidobacterium breve, Bifidobacterium catenulatum, Bifidobacterium dentium, Bifidobacterium eriksonii, Bifidobacterium infantis, Bifidobacterium longum, Bifidobacterium plantarum, Bifidobacterium pseudocatenulatum, Bifidobacterium pseudolongum, Streptococcus lactis, Streptococcus raffinolactis and Streptococcus thermophilus.*

7. Composition according to claim 6, **characterized in that** the *Lactobacillus brevis* CD2 strain is used, which is filed on February 6, 1998 under access No. DSM 11,988 in the German Collection of Microorganisms and Cell Cultures (DSM) in Braunschweig, Germany ("Deutsche Sammlung von Mikroorganismen und Zellkulturen GmbH"), or mutants or derivatives thereof under the Budapest Treaty.

8. Composition according to any one of the preceding claims, **characterized in that** the lactic acid bacteria are used in the composition as live, lyophilized or sonicated bacteria.

9. Composition according to any one of the preceding claims, **characterized in that** it contains from 1x10² to 1x10¹³ CFUs of lactic acid bacteria per gram of composition.

10. Composition according to any one of claims 6 to 8, **characterized in that** it contains 200x10⁹ *Streptococcus thermophilus,* 150x10⁹ *Bifidobacteria* and 4x10⁹ *Lactobacillus acidophilus* per gram of composition.

11. Composition according to any one of the preceding claims, **characterized in that** it also contains bile acids, in particular ursodeoxycholic acid, pectin, sphingomyelin or its compounds, drugs or foods containing sphingomyelin, arginine deiminase, fatty acids, polyunsaturated fatty acids, non fermented sugars, in particular lactulose, cholesterol inhibitors, ceramidase inhibitors, protease inhibitors, immunomodulators, anti-carcinogenic agents, vitamins, growth factors, surfactants, cereals, fibre, emulsifiers, stabilizers, lipids, antioxidants, preservatives, free-radical neutralizers and/or vasoprotectors.

12. Composition according to any one of the preceding claims, which can be administered orally as a food supplement.

13. Composition according to any one of the preceding claims, which can be administered orally or parenterally as a drug.

14. Use of alkaline sphingomyelinase of bacterial origin, and the bacteria containing the alkaline sphingomyelinase are chosen from amongst Gram-positive bacteria, Gram-negative bacteria and lactic acid bacteria, or from mixtures thereof, for the preparation of a dietetic, nutrient or pharmaceutical composition suitable for the prevention and/or treatment of disorders connected with intestinal development, cancerous processes, disorders of the immune response, inflammatory and apoptosic processes of the intestine and its associated structures, disorders connected with cholesterol synthesis, disorders due to the hydrophobic nature of the surfaces of the gastrointestinal tract, allergic disorders of the gastrointestinal tract, disorders relating to digestive processes, inflammatory intestinal diseases, polyposis, in particular familial polyposis, hypercholesterolaemia, infections with *Helicobacter pylori,* disorders of neonatal growth, disorders connected with intestinal homeostasis and diseases of the central and peripheral nervous systems.

15. Use according to claim 14 in pediatric diets.

16. Use according to claim 14, in enteral alimentation.

17. Use according to claim 15, **characterized in that** the composition is administered, in combination with artificial milk, condensed milk, soybean milk, powdered milk, partially humanized milk and baby foods in general.

18. Use according to any one of claims 14-17, **characterized in that** the lactic acid bacteria are chosen from the group comprising *Lactobacillus acidophilus, Lactobacillus brevis, Lactobacillus buchneri, Lactobacillus casei, Lactobacillus catenaforme, Lactobacillus cellobiosus, Lactobacillus crispatus, Lactobacillus curvatus, Lactobacillus delbrueckii, Lactobacillus fermentum, Lactobacillus jensenii, Lactobacillus leichmannii, Lactobacillus, minutus, Lactobacillus plantarum, Lactobacillus rogosae, Lactobacillus saliuarius, Bifidobacterium adolescentis, Bifidobacterium angulatum, Bifidobacterium bifidum, Bifidobacterium breve, Bifidobacterium catenulatum, Bifidobacterium dentium, Bifidobacterium eriksonii, Bifidobacterium infantis, Bifidobacterium longum, Bifidobacterium plantarum, Bifidobacterium pseudocatenulatum, Bifidobacterium pseudolongum, Streptococcus lactis, Streptococcus raffinolactis* and *Streptococcus thermophilus.*

19. Use according to claim 18, **characterized in that** the *Lactobacillus brevis* CD2 strain is used, which is filed on February 6, 1998 under access No. DSM 11,988 in the German Collection of Microorganisms and Cell Cultures (DSM) in Braunschweig, Germany ("Deutsche Sammlung von Mikroorganismen und Zellkulturen GmbH") under the Budapest Treaty, or mutants or derivatives thereof.

20. Use according to any one of claims 14 to 19, **characterized in that** the lactic acid bacteria are used in the composition as live, lyophilized or sonicated bacteria.

21. Use according to any one of claims 14 to 19, **characterized in that** from 1x10² to 1x10¹³ CFUs of lactic acid bacteria are used per gram of composition.

22. Use according to any one of claims 14 to 19, **characterized in that** 200x10² *Streptococcus thermophilus,* 150x10⁹ *Bifidobacteria* and 4x10⁹ *Lactobacillus acidophilus* are used per gram of composition.

## Patentansprüche

1. Diätetische, Nahrungs- oder pharmazeutische Zusammensetzung, **dadurch gekennzeichnet, daß** sie alkalische Sphingomyelinase von bakteriellem Ursprung, wobei die Bakterien, die die alkalische Sphingomyelinase enthalten, ausgewählt sind aus gram-positiven Bakterien, gram-negativen Bakterien und Milchsäurebakterien und Mischungen davon, in einer ausreichenden Menge umfaßt, um eine diätetische, Nahrungs- oder therapeutische Wirkung bei einem bedürftigen Individuum auszuüben.

2. Zusammensetzung gemäß Anspruch 1, geeignet zur Verhinderung und/ oder Behandlung von Störungen, die in Zusammenhang mit der Darmentwicklung, krebsartigen Abläufen, Störungen der Immunreaktion, entzündlichen und Zelltod-Abläufen des Darms und seiner assoziierten Strukturen, Störungen in Verbindung mit der Cholesterinsynthese, Störungen aufgrund der hydrophoben Natur von Oberflächen des Gastrointestinaltrakts, allergischen Störungen des Gastrointestinaltrakts, Störungen in Bezug auf Verdauungsabläufe, entzündliche Darmerkrankungen, Polypenbildung, insbesondere familiäre Polypenbildung, Hypercholesterinämie, Infektionen mit Helicobacter pylori, Störungen des Neonatalwachstums, Störungen in Verbindung mit der Darmhomöostase und Krankheiten des zentralen und peripheren Nervensystems stehen.

3. Zusammensetzung gemäß Anspruch 1 oder 2, geeignet zur Verwendung bei pädiatrischen Diäten.

4. Zusammensetzung gemäß Anspruch 1 oder 2, geeignet zur Verwendung bei einer enteralen Ernährung.

5. Zusammensetzung gemäß Anspruch 3, **dadurch gekennzeichnet, daß** die Zusammensetzung zusammen mit künstlicher Milch, Kondensmilch, Sojamilch, pulverförmiger Milch, teilweise humanisierter Milch und Babyernährung im allgemeinen verabreicht wird.

6. Zusammensetzung gemäß einem der vorstehenden Ansprüche, **dadurch gekennzeichnet, daß** die Milchsäurebakterien ausgewählt werden aus der Gruppe umfassend Lactobacillus acidophilus, Lactobacillus brevis, Lactobacillus buchneri, Lactobacillus casei, Lactobacillus catenaforme, Lactobacillus cellobiosus, Lactobacillus crispatus, Lactobacillus curvatus, Lactobacillus delbrueckii, Lactobacillus fermentum, Lactobacillus jensenii, Lactobacillus leichmannii, Lactobacillus minutus, Lactobacillus plantarum, Lactobacillus rogosae, Lactobacillus salivarius, Bifidobacterium adolescentis, Bifidobacterium angulatum, Bifidobacterium bifidum, Bifidobacterium breve, Bifidobacterium catenulatum, Bifidobacterium dentium, Bifidobacterium eriksonii, Bifidobacterium infantis, Bifidobacterium longum, Bifidobacterium plantarum, Bifidobacterium pseudocatenulatum, Bifidobacterium pseudolongum, Streptococcus lactis, Streptococcus raffinolactis und Streptococcus thermophilus.

7. Zusammensetzung gemäß Anspruch 6, **dadurch gekennzeichnet, daß** der Lactobacillus brevis CD2 Stamm verwendet wird, der am 6. Februar 1998 unter der Hinterlegungsnr. DSM 11,988 bei der Deutschen Sammlung für Mikroorganismen und Zellkulturen (DSM) in Braunschweig, Deutschland gemäß dem Budapester Vertrag hinterlegt wurde, oder Mutanten oder Derivate davon.

8. Zusammensetzung gemäß einem der vorstehenden Ansprüche, **dadurch gekennzeichnet, daß** die Milchsäurebakterien in der Zusammensetzung als lebende, lyophilisierte oder beschallte Bakterien verwendet werden.

9. Zusammensetzung gemäß einem der vorstehenden Ansprüche, **dadurch gekennzeichnet, daß** sie 1 x 10² bis 1 x 10¹³ CBUs Milchsäurebakterien pro Gramm der Zusammensetzung enthält.

10. Zusammensetzung gemäß einem der Ansprüche 6 bis 8, **dadurch gekennzeichnet, daß** sie 200 x 10⁹ Streptococcus thermophilus, 150 x 10⁹ Bifidobakterien und 4 x 10⁹ Lactobacillus acidophilus pro Gramm der Zusammensetzung enthält.

11. Zusammensetzung gemäß einem der vorstehenden Ansprüche, **dadurch gekennzeichnet, daß** sie außerdem Gallensäuren, insbesondere Ursodeoxycholinsäure, Pektin, Sphingomyelin oder ihre Verbindungen, Arzneimittel oder Nahrungsmittel enthält, enthaltend Sphingomyelin, Arginindeaminase, Fettsäuren, mehrfach ungesättigte Fettsäuren, nicht fermentierte Zucker, insbesondere Lactulose, Cholesterininhibitoren, Ceramidaseinhibitoren, Proteaseinhibitoren, Immunmodulatoren, Antitumormittel, Vitamine, Wachstumsfaktoren, Tenside, Getreide, Fasern, Emulgatoren, Stabilisatoren, Lipide, Antioxidanzien, Konservierungsmittel, Neutralisierungsmittel für freie Radikale und/oder Vasoprotektoren.

12. Zusammensetzung gemäß einem der vorstehenden Ansprüche, die oral als Nahrungsmittelergänzung verabreicht werden kann.

13. Zusammensetzung gemäß einem der vorstehenden Ansprüche, die oral oder parenteral als Arzneimittel verabreicht werden kann.

14. Verwendung der alkalischen Sphingomyelinase von bakteriellem Ursprung, wobei die Bakterien, die die alkalische Sphingomyelinase enthalten, ausgewählt sind aus gram-positiven Bakterien, gram-negativen Bakterien und Milchsäurebakterien und Mischungen davon, für die Herstellung einer diätetischen, Nahrungs- oder pharmazeutischen Zusammensetzung, geeignet für die Verhinderung und/oder Behandlung von Störungen, die in Zusammenhang mit der Darmentwicklung, krebsartigen Abläufen, Störungen der Immunreaktion, entzündlichen und Zelltod-Abläufen des Darms und seiner assoziierten Strukturen, Störungen in Verbindung mit der Cholesterinsynthese, Störungen aufgrund der hydrophoben Natur von Oberflächen des Gastrointestinaltrakts, allergischen Störungen des Gastrointestinaltrakts, Störungen in Bezug auf Verdauungsabläufe, entzündliche Darmerkrankungen, Polypenbildung, insbesondere familiäre Polypenbildung, Hypercholesterolämie, Infektionen mit Helicobacter pylori, Störungen des Neonatalwachstums, Störungen in Verbindung mit der Darmhomöostase und Krankheiten des zentralen und peripheren Nervensystems stehen.

15. Verwendung gemäß Anspruch 14 in pädiatrischen Diäten.

16. Verwendung gemäß Anspruch 14 bei enteraler Ernährung.

17. Verwendung gemäß Anspruch 15, **dadurch gekennzeichnet, daß** die Zusammensetzung zusammen mit künstlicher Milch, Kondensmilch, Sojamilch, pulverförmiger Milch, teilweise humanisierter Milch und Babynahrung im allgemeinen verabreicht wird.

18. Verwendung gemäß einem der Ansprüche 14 bis 17, **dadurch gekennzeichnet, daß** die Milchsäurebakterien ausgewählt werden aus der Gruppe umfassend Lactobacillus acidophilus, Lactobacillus brevis, Lactobacillus buchneri, Lactobacillus casei, Lactobacillus catenaforme, Lactobacillus cellobiosus, Lactobacillus crispatus, Lactobacillus curvatus, Lactobacillus delbrueckii, Lactobacillus fermentum, Lactobacillus jensenii, Lactobacillus leichmannii, Lactobacillus minutus, Lactobacillus plantarum, Lactobacillus rogosae, Lactobacillus salivarius, Bifidobacterium adolescentis, Bifidobacterium angulatum, Bifidobacterium bifidum, Bifidobacterium breve, Bifidobacterium catenulatum, Bifidobacterium dentium, Bifidobacterium eriksonii, Bifidobacterium infantis, Bifidobacterium longum, Bifidobacterium plantarum, Bifidobacterium pseudocatenulatum, Bifidobacterium pseudolongum, Streptococcus lactis, Streptococcus raffinolactis und Streptococcus thermophilus.

19. Verwendung gemäß Anspruch 18, **dadurch gekennzeichnet, daß** der Lactobacillus brevis CD2 Stamm verwendet wird, der am 6. Februar 1998 unter der Hinterlegungsnr. DSM 11,988 bei der Deutschen Sammlung für Mikroorganismen und Zellkulturen (DSM) in Braunschweig, Deutschland gemäß dem Budapester Vertrag hinterlegt wurde, oder Mutanten oder Derivate davon.

20. Verwendung gemäß einem der Ansprüche 14 bis 19, **dadurch gekennzeichnet, daß** die Milchsäurebakterien in der Zusammensetzung als lebende, lyophilisierte oder beschallte Bakterien verwendet werden.

21. Verwendung gemäß einem der Ansprüche 14 bis 19, **dadurch gekennzeichnet, daß** sie 1 x 10² bis 1 x 10¹³ CBUs Milchsäurebakterien pro Gramm der Zusammensetzung enthält.

22. Verwendung gemäß einem der Ansprüche 14 bis 19, **dadurch gekennzeichnet, daß** sie 200 x 10⁹ Streptococcus thermophilus, 150 x 10⁹ Bifidobakterien und 4 x 10⁹ Lactobacillus acidophilus pro Gramm der Zusammensetzung enthält.

## Revendications

1. Composition diététique, nutritive ou pharmaceutique **caractérisée en ce qu'**elle comprend de la sphingomyélinase alcaline d'origine bactérienne, et les bactéries contenant la sphingomyélinase alcaline sont choisies parmi les bactéries Gram-positives, les bactéries Gram-négatives et les bactéries lactiques, ou des mélanges de celles-ci ; en quantité suffisante pour exercer un effet diététique, nutritionnel ou thérapeutique chez un individu en ayant besoin.

2. Composition selon la revendication 1, convenant pour la prévention et/ou le traitement de troubles liés au développement intestinal, à des processus cancéreux, à des troubles de la réponse immunitaire, aux processus inflammatoires et apoptotiques de l'intestin et de ses structures associées, aux troubles liés à la synthèse du cholestérol, aux troubles dus à la nature hydrophobe des surfaces du tractus gastro-intestinal, aux troubles allergiques du tractus gastro-intestinal, aux troubles liés aux processus digestifs, aux maladies intestinales inflammatoires, aux polyposes, en particulier aux polyposes familiales, à l'hypercholestérolémic, aux infections avec *Helicobacter pylori,* aux troubles de la croissance néonatale, aux troubles liés à l'homéostasie intestinale et aux maladies des systèmes nerveux central et périphérique.

3. Composition selon la revendication 1 ou 2, convenant à une utilisation dans des régimes pédiatriques.

4. Composition selon la revendication 1 ou 2, convenant à une utilisation en alimentation entérale.

5. Composition selon la revendication 3, **caractérisée en ce que** la composition est administrée en combinaison avec du lait artificiel, du lait concentré, du lait de soja, du lait en poudre, du lait partiellement humanisé et des aliments pour bébé en général.

6. Composition selon l'une quelconque des revendications précédentes, **caractérisée en ce que** les bactéries lactiques sont choisies dans le groupe comprenant *Lactobacillus acidophilus, Lactobacillus brevis, Lactobacillus buchneri, Lactobacillus casei, Lactobacillus catenaforme, Lactobacillus cellobiosus, Lactobacillus crispactus, Lactobacillus curvarus, Lactobacillus delbrueckii, Lactobacillus fermentum, Lactobacillus jensenii, Lactobacillus leichmannii, Lactobacillus minutus, Lactobacillus plantarum, Lactobacillus rogosae, Lactobacillus salivarius, Bifidobacterium adolescentis, Bifidobacterium angulatum, Bifidobacterium bifidum, Bifidobacterium breve, Bifidobacterium catenulatum, Bifidobacterium dentium, Bifidobacterium eriksonii, Bifidobacterium infantis, Bifidobacterium longum, Bifidobacterium plantarum, Bifidobacterium pseudocatenulatum, Bifidobacterium pseudolongum, Streptococcus lactis, Streptococcus raffinolactis* et *Streptococcus thermophilus.*

7. Composition selon la revendication 6, **caractérisée en ce qu'**est utilisée la souche *Lactobacillus brevis* CD2, qui a été déposée le 6 février 1998 sous le numéro d'accès DSM 11 988 dans la Collection Allemande de Micro-organismes et de Cultures Cellulaires (DSM) à Braunschweig, Allemagne (« Deutsche Sammlung von Mikroorganismen und Zellkulturen GmbH »), ou des mutants ou dérivés de celle-ci selon le Traité de Budapest.

8. Composition selon l'une quelconque des revendications précédentes, **caractérisée en ce que** les bactéries lactiques sont utilisées dans la composition en tant que bactéries vivantes, lyophilisées ou soniquées.

9. Composition selon l'une quelconque des revendications précédentes, **caractérisée en ce que**lle contient de 1 x 10² à 1 x 10¹³ UFC de bactéries lactiques par gramme de composition.

10. Composition selon l'une des revendication 6 à 8, **caractérisée en ce qu'**elle contient 200 x 10⁹ *Streptococcus thermophilus,* 150 x 10⁹ Bifidobactéries et 4 x 10⁹ *Lactobacillus acidophilus* par gramme de composition.

11. Composition selon l'une quelconque des revendications précédentes, **caractérisée en ce qu'**elle contient également des acides biliaires, en particulier de l'acide ursodéoxycholique, de la pectine, de la sphingomyéline ou ses composés, des médicaments ou des aliments contenant de la sphingomyéline, de l'arginine déiminase, des acides gras, des acides gras poly-insaturés, des sucres non-fermentés, en particulier du lactulose, des inhibiteurs du cholestérol, des inhibiteurs de céramidases, des inhibiteurs de protéases, des immuno-modulateurs, des agents anti-cancérogènes, des vitamines, des facteurs de croissance, des surfactants, des céréales, des fibres, des émulsifiants, des stabilisants, des lipides, des antioxydants, des conservateurs, des neutralisateurs de radicaux libres et/ou des vasoprotecteurs.

12. Composition selon l'une quelconque des revendications précédentes, qui peut être administrée par voie orale en tant que supplément alimentaire.

13. Composition selon l'une quelconque des revendications précédentes, qui peut être administrée par voie orale ou parentérale en tant que médicament.

14. Utilisation de la sphingomyélinase alcaline d'origine bactérienne, et les bactéries contenant la shingomyélinase alcaline sont choisies parmi les bactéries Gram-positives, les bactéries Gram-négatives et les bactéries lactiques, ou des mélanges de celles-ci, pour la préparation d'une composition diététique, nutritive ou pharmaceutique convenant pour la prévention et/ou le traitement de troubles liés au développement intestinal, à des processus cancéreux, à des troubles de la réponse immunitaire, aux processus inflammatoires et apoptotiques de l'intestin et de ses structures associées, aux troubles liés à la synthèse du cholestérol, aux troubles dus à la nature hydrophobe des surfaces du tractus gastro-intestinal, aux troubles allergiques du tractus gastro-intestinal, aux troubles liés aux processus digestifs, aux maladies intestinales inflammatoires, aux polyposes, en particulier les polyposes familiales, à l'hypercholestérolémie, aux infections avec *Helicobacter pylori,* aux troubles de la croissance néonatale, aux troubles liés à l'homéostasie intestinale et aux maladies des systèmes nerveux central et périphérique.

15. Utilisation selon la revendication 14 dans des régimes pédiatriques.

16. Utilisation selon la revendication 14, en alimentation entérale.

17. Utilisation selon la revendication 15, **caractérisée en ce que** la composition est administrée, en combinaison avec du lait artificiel, du lait concentré, du lait de soja, du lait en poudre, du lait partiellement humanisé et des aliments pour bébé en général.

18. Utilisation selon l'une quelconque des revendications 14 à 17, **caractérisée en ce que** les bactéries lactiques sont choisies dans le groupe comprenant *Lactobacillus acidophilus, Lactobacillus brevis, Lactobacillus buchneri, Lactobacillus casei, Lactobacillus catenaforme, Lactobacillus cellobiosus, Lactobacillus crispatus, Lactobacillus curvatus, Lactobacillus delbrueckii, Lactobacillus fermentum, Lactobacillus jensenii, Lactobacillus leichmannii, Lactobacillus minutus, Lactobacillus plantarum, Lactobacillus rogosae, Lactobacillus salivarius, Bifidobacterium adolescentis, Bifidobacterium angulatum, Bifidobacterium bifidum, Bifidobacterium breve, Bifidobacterium catenulatum, Bifidobacterium dentium, Bifidobacterium eriksonii, Bifidobacterium infantis, Bifidobacterium longum, Bifidobacterium plantarum, Bifidobacterium pseudocatenulatum, Bifidobacterium pseudolongum, Streptococcus lactis, Streptococcus raffinolactis* et *Streptococcus thermophilus.*

19. Utilisation selon la revendication 18, **caractérisée en ce qu'**est utilisée la souche *Lactobacillus brevis* CD2, qui a été déposée le 6 février 1998 sous le numéro d'accès DSM 11 988 dans la Collection Allemande de Micro-organismes et de Cultures Cellulaires (DSM) à Braunschweig, Allemagne (« Deutsche Sammlung von Mikroorganismen und Zellkulturen GmbH ») selon le Traité de Budapest, ou des mutants ou des dérivés de celle-ci.

20. Utilisation selon l'une quelconque des revendications 14 à 19, **caractérisée en ce que** les bactéries lactiques sont utilisées dans la composition en tant que bactéries vivantes, lyophilisées ou soniquées.

21. Utilisation selon l'une quelconque des revendications 14 à 19, **caractérisée en ce que** de 1 x 10² à 1 x 10¹³ UFC de bactéries lactiques sont utilisées par gramme de composition.

22. Utilisation selon l'une quelconque des revendications 14 à 19, **caractérisée en ce que** 200 x 10⁹ *Streptococcus thermophilus,* 150 x 10⁹ Bifidobactéries et 4 x 10⁹ *Lactobacillus acidophilus* sont utilisés par gramme de composition.
